# EUROPEAN PATENT SPECIFICATION

(11) **EP 2 621 920 B1**
(45) Date of publication and mention of the grant of the patent: **27.01.2016**
(21) Application number: 11764930.1
(22) Date of filing: 23.09.2011
(51) Int. Cl.: C07D 403/06, C07D 413/06, C07D 413/14, A61K 31/496, A61P 31/12, A61P 31/16

(54) **NOVEL PIPERAZINE ANALOGS WITH SUBSTITUTED HETEROARYL GROUPS AS BROAD-SPECTRUM INFLUENZA ANTIVIRALS**
NEUE PIPERAZINANALOGA MIT SUBSTITUIERTEN HETEROARYLGRUPPEN ALS BREITSPEKTRUM-INFLUENAZVIRUZIDE
NOUVEAUX ANALOGUES DE LA PIPÉRAZINE COMPORTANT DES GROUPES HÉTÉROARYLE SUBSTITUÉS UTILISÉS COMME ANTIVIRAUX À LARGE SPECTRE PROTÉGEANT DE LA GRIPPE

(30) Priority: 28.09.2010 US 387186 P
(43) Date of publication of application: 07.08.2013
(73) Proprietor: Bristol-Myers Squibb Company, Princeton, NJ 08543 (US)
(72) Inventor: CIANCI, Christopher W., Wallingford, Connecticut 06492 (US); GERRITZ,Samuel, Wallingford, Connecticut 06492 (US); KIM, Sean, Acton, Massachusetts 01720 (US); LANGLEY, David R., Wallingford, Connecticut 06492 (US); LI, Guo, Wallingford, Connecticut 06492 (US); PEARCE, Bradley C., Wallingford, Connecticut 06492 (US); PENDRI, Annapurna, Wallingford, Connecticut 06492 (US); SHI, Shuhao, Wallingford, Connecticut 06492 (US); ZHAI, Weixu, Wallingford, Connecticut 06492 (US); ZHU, Shirong, Wallingford, Connecticut 06492 (US)
(74) Representative: Beacham, Annabel Rose
(86) International application number: PCT/US2011/052965
(87) International publication number: WO 2012/044531

(56) References cited:
- EP-A1- 1 396 487
- WO-A1-2011/015037
- WO-A2-2009/131957
- C.-Y. SU ET AL: "High-throughput identification of compounds targeting influenza RNA-dependent RNA polymerase activity", PROCEEDINGS OF THE NATIONAL ACADEMY OF SCIENCES, vol. 107, no. 45, 9 November 2010 (2010-11-09), pages 19151-19156, XP55009856, ISSN: 0027-8424, DOI: 10.1073/pnas.1013592107

## Description

### FIELD OF THE INVENTION

The present invention relates to novel piperazine compounds with one or more substituted heteroaryl groups, useful for the prophylaxis and treatment of influenza virus, and to compositions and formulations containing these compounds.

### BACKGROUND OF THE INVENTION

Influenza virus is a significant causative agent of acute lower respiratory tract infections in humans. It transmits readily, resulting in annual epidemics that can manifest in severe illness and death for high-risk populations. It is one of the RNA viruses of the family *Orthomyxoviridae* that affects birds and mammals, and is responsible for the illness commonly referred to as the "flu". The most common symptoms of the flu are chills, fever, sore throat, muscle pains, severe headache, coughing, weakness/fatigue and general discomfort. Sore throat, fever and coughs are the most frequent symptoms. In more serious cases, influenza causes pneumonia, which can be fatal, particularly for the young and the elderly. Although it is often confused with other influenza-like illnesses, especially the common cold, influenza is a more severe disease than the common cold and is caused by a different type of virus. Influenza may produce nausea and vomiting, particularly in children, but these symptoms are more common in the unrelated gastroenteritis, which is sometimes called "stomach flu" or "24-hour flu".

Typically, the influenza virus is transmitted through the air by coughs or sneezes, creating aerosols containing the virus. Influenza can also be transmitted by direct contact with bird droppings or nasal secretions, or through contact with contaminated surfaces. Airborne aerosols have been thought to cause most infections, although which means of transmission is most important is not absolutely clear.

Influenza remains a constant threat, as new variants emerge seasonally. Annual epidemics take an economic toll through lost workforce productivity, while straining health service resources. Additionally, influenza virus is responsible for major pandemics every 10-50 years. In 2009, a new H1N1 triple re-assortment of swine influenza emerged in North America and reached pandemic proportion (Zimmer and Burke, 2009). Influenza virus' ability to mutate (antigenic drift), as well as re-assort with other influenza viruses from different mammalian species (antigenic shift), are mechanisms causing seasonal epidemic variation and pandemic virus insurgence, respectively (Chen and Deng, 2009). Moreover, resistance to available anti-influenza agents is increasing. The majority of H3N2 isolates and 2009 H1N1 are resistant to the adamantane M2 ion channel inhibitors (Deyde et al, 2009). Furthermore, 2008 H1N1 has shown resistance to the neuraminidase inhibitor Tamiflu (Oseltamivir), the standard of care (Moscona, 2009). Neither class has been shown to be effective against highly pathogenic H5N1 avian virus (Soepandi, 2010).

WO 2011/015037 discloses compounds having antiviral activity. In particular, this document discloses heterocyclic amides containing piperazine and isozazole rings that may be useful in the treatment of influenza. Su et al. (PNAS, 2010, vol 7, no. 45, 19151-19156) discusses a screening procedure developed to search for new anti influenza inhibitors. WO 2009/131957 discloses compositions and methods for treating a disease in a mammal that is responsive to inhibition of CFTR polypeptide, such as diarrhea and polycystic kidney disease. EP 1396487 A1 discloses compounds that are type 4 phosphodiesterase inhibitors.

Multiple novel therapeutic and prophylactic agents against influenza virus are therefore currently needed in the art. Also needed are new compositions and formulations containing these agents.

### SUMMARY OF THE INVENTION

The invention provides compounds that are selected from the group consisting of: 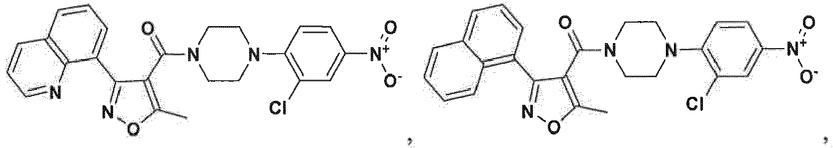 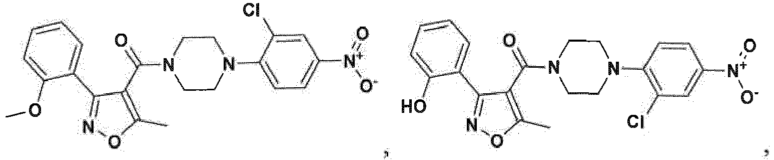 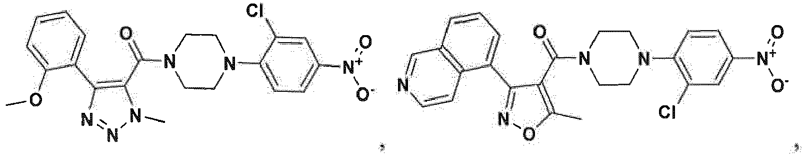 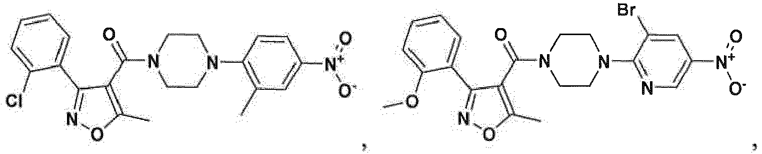 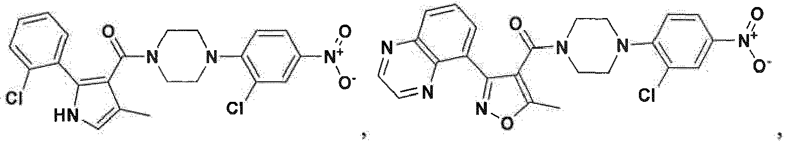 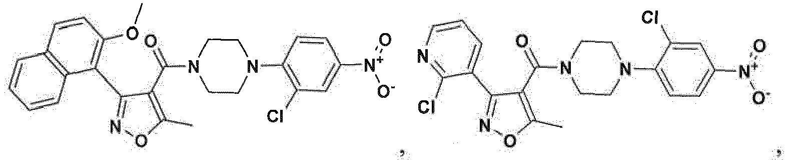 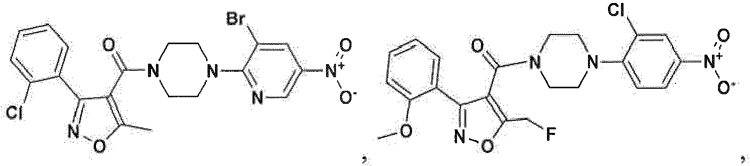 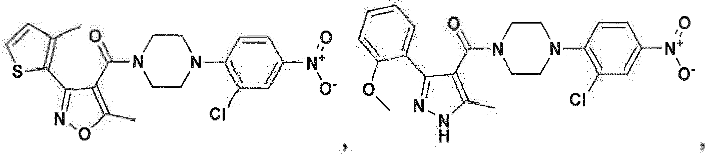 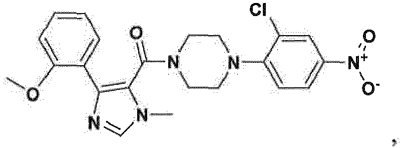 and pharmaceutically acceptable salts thereof.

Also provided as part of the invention is a pharmaceutical composition which comprises an antiviral effective amount of one or more of the compounds of the invention, including pharmaceutically acceptable salts thereof, together with one or more pharmaceutically acceptable carriers, excipients or diluents.

In addition, there is provided a compound of the invention including pharmaceutically acceptable salts thereof, and one or more pharmaceutically acceptable carriers, excipients or diluents for use in treating a mammal infected with influenza virus.

Methods for making the compounds of the invention are also herein provided.

The invention is directed to these and other important ends, hereinafter described.

### DETAILED DESCRIPTION OF THE EMBODIMENTS

Since the compounds of the present invention may possess asymmetric centers and therefore occur as mixtures of diastereomers and enantiomers, the present invention includes the individual diastereoisomeric and enantiomeric forms of the compounds of the invention in addition to the mixtures thereof.

### Definitions

Unless otherwise specifically set forth elsewhere in the application, one or more of the following terms may be used herein, and shall have the following meanings:
It is known in the art that nitrogen atoms in heteroaryl systems can be "participating in a heteroaryl ring double bond", and this refers to the form of double bonds in the two tautomeric structures which comprise five-member ring heteroaryl groups. This dictates whether nitrogens can be substituted as well understood by chemists in the art. The disclosure and claims of the present disclosure are based on the known general principles of chemical bonding. It is understood that the claims do not encompass structures known to be unstable or not able to exist based on the literature.

Physiologically acceptable salts of compounds disclosed herein are within the scope of this disclosure. The term "pharmaceutically acceptable salt" as used herein and in the claims is intended to include nontoxic base addition salts. Suitable salts include those derived from organic and inorganic acids such as, without limitation, hydrochloric acid, hydrobromic acid, phosphoric acid, sulfuric acid, methanesulfonic acid, acetic acid, tartaric acid, lactic acid, sulfinic acid, citric acid, maleic acid, fumaric acid, sorbic acid, aconitic acid, salicylic acid, phthalic acid, and the like. The term "pharmaceutically acceptable salt" as used herein is also intended to include salts of acidic groups, such as a carboxylate, with such counterions as ammonium, alkali metal salts, particularly sodium or potassium, alkaline earth metal salts, particularly calcium or magnesium, and salts with suitable organic bases such as lower alkylamines (methylamine, ethylamine, cyclohexylamine, and the like) or with substituted lower alkylamines (e.g. hydroxyl-substituted alkylamines such as diethanolamine, triethanolamine or tris(hydroxymethyl)-aminomethane), or with bases such as piperidine or morpholine.

As set forth above, the present invention is directed to the specific compounds below, including pharmaceutically acceptable salts thereof: 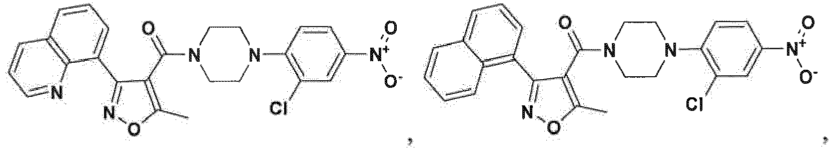 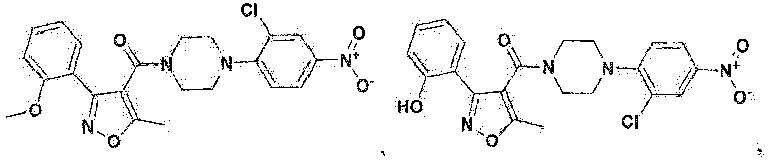 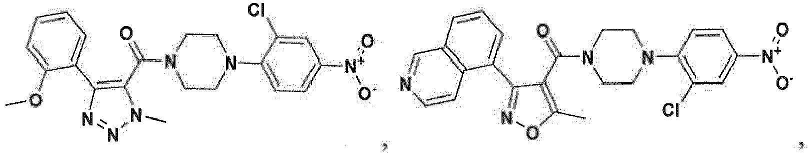 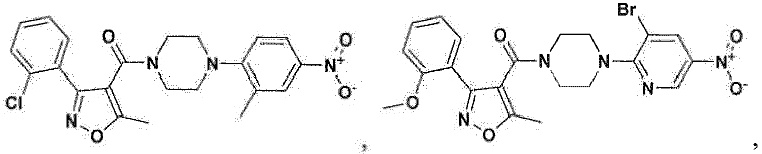 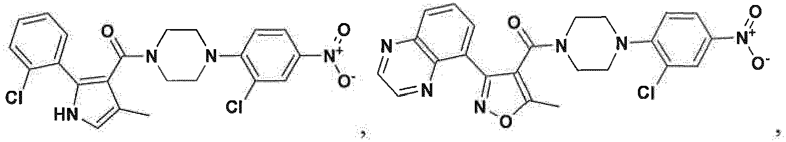 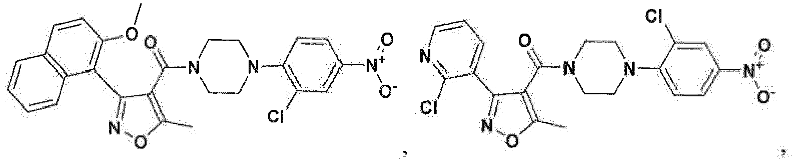 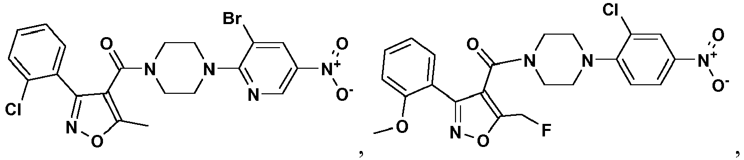 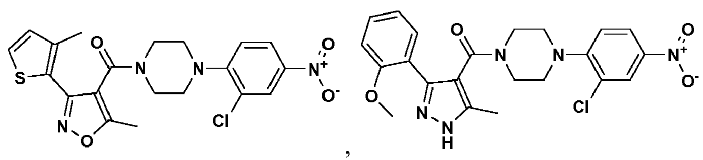 and

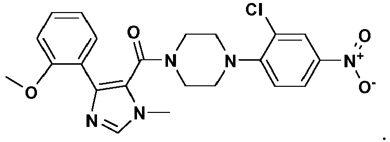

The compounds of the present invention may be administered orally, parenterally (including subcutaneous injections, intravenous, intramuscular, intrasternal injection or infusion techniques), by inhalation spray, or rectally or by other means available in the art, in dosage unit formulations containing conventional non-toxic pharmaceutically acceptable carriers, adjuvants and diluents.

Thus, in accordance with the present invention, there is further provided a pharmaceutical composition comprising a pharmaceutical carrier and a therapeutically effective amount of a compound of the present disclosure for use in treating viral infections such as influenza infection.

The pharmaceutical composition may be in the form of orally administrable suspensions or tablets; nasal sprays, sterile injectable preparations, for example, as sterile injectable aqueous or oleaginous suspensions or suppositories.

When administered orally as a suspension, these compositions are prepared according to techniques available in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients, binders, extenders, disintegrants, diluents, and lubricants known in the art.

The injectable solutions or suspensions may be formulated according to known art, using suitable non-toxic, parenterally acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

The compounds herein set forth can be administered orally to humans in a dosage range of 1 to 100 mg/kg body weight, perhaps in divided doses. One preferred dosage range is 1 to 10 mg/kg body weight orally in divided doses. Another preferred dosage range is 1 to 20 mg/kg body weight in divided doses. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The term "antiviral effective amount" means the total amount of each active compound or component of the composition or method that is sufficient to show a meaningful patient benefit, e.g., prevention of infection by influenza or healing of acute conditions or symptoms characterized by influenza infection. The terms "treat, treating, treatment" as used herein and in the claims means preventing or ameliorating diseases and symptoms associated with influenza infection. When applied to an individual active ingredient, administered alone, the term refers to that ingredient alone. When applied to a combination, the term refers to combined amounts of the active ingredients that result in the therapeutic effect, whether administered in combination, serially or simultaneously.

The present invention is also directed to combinations of the compounds herein described with one or more other agents useful in the treatment of influenza. For example, the compounds of this invention may be effectively administered, whether at periods of pre-exposure and/or post-exposure, in combination with effective amounts of other influenza antivirals, immunomodulators, antiinfectives, or vaccines available in the art.

The following schemata are generalized procedures for making the compounds of the invention by those skilled in the art. 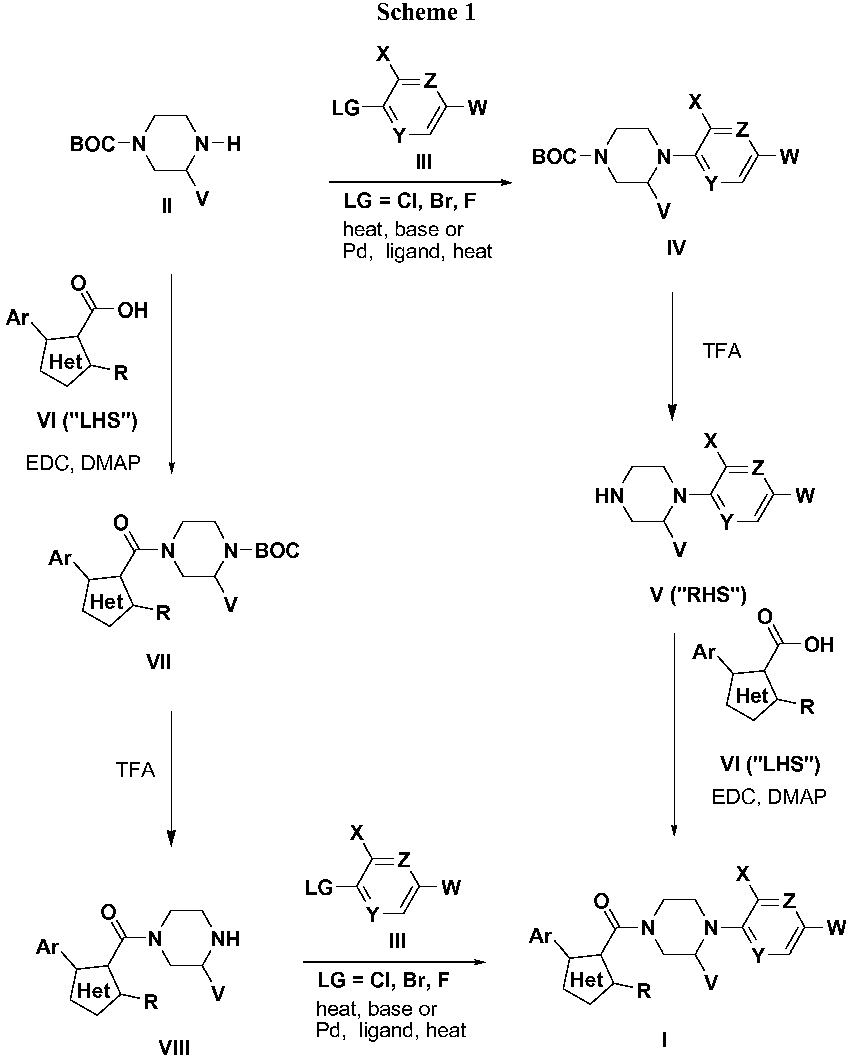 wherein V is H, X is Cl, Br or Me, Y is CH or N, Z is CH, W is NO₂, R is Me or CH₂F and Het and Ar are the aromatic groups shown in claim 1. Compounds of formula **I** were prepared from intermediates of formula **II** via two complementary routes as illustrated in Scheme 1. In the first route, intermediates of formula **II** were treated with intermediates of formula **III** in the presence of base and heat or in the presence of base, heat and a palladium catalyst to afford intermediates of formula **IV**. Intermediates of formula **III** can be obtained commercially, can be prepared by methods known in the literature, or can be readily prepared by one skilled in the art. Intermediates of formula **IV** were treated with TFA to provide intermediates of formula **V** (also referred to as the "RHS" or right hand side). Intermediates of formula **V** were treated with carboxylic acids of formula **VI** (also referred to as the "LHS" or left hand side) and an amide-bond forming reagent (i.e. EDC) to provide compounds of formula **I**. Carboxylic acids of formula **IV** can be obtained commercially, can be prepared by methods known in the literature, or can be readily prepared by one skilled in the art. In the second route, the synthetic steps described in the first route are reversed. Intermediates of formula **II** were treated with carboxylic acids of formula **VI** (also referred to as the "LHS" or left hand side) and an amide-bond forming reagent to afford intermediates of formula **VII**. Carboxylic acids of formula **VI** can be obtained commercially, can be prepared by methods known in the literature, or can be readily prepared by one skilled in the art. Intermediates of formula **VII** were treated with trifluoroacetic acid to afford intermediates of formula **VIII**. Intermediates of formula **VIII** were treated with intermediates of formula **III** in the presence of base and heat or in the presence of base, heat and a palladium catalyst to afford compounds of formula **I**. Intermediates of formula **III** can be obtained commercially, can be prepared by methods known in the literature, or can be readily prepared by one skilled in the art.

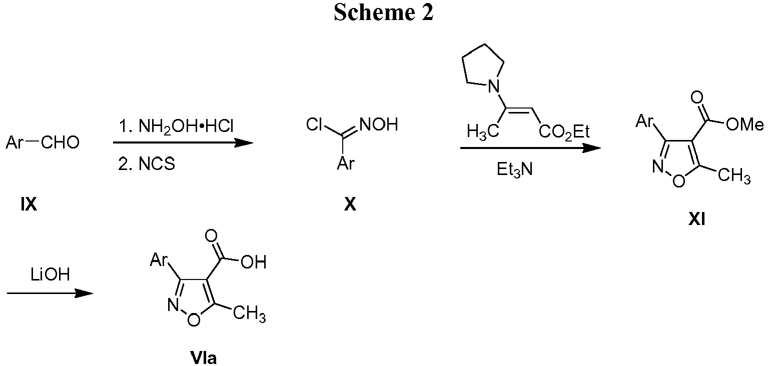

Compounds of formula **VIa** were prepared as outlined in Scheme 2 and as described in the literature. [See: Gerald W.Zamponi, Stephanie C. Stotz, Richard J. Staples, Tina M. Andro, Jared K. Nelson, Victoria Hulubei, Alex Blumenfeld, and Nicholas R. Natale, J. Med. Chem., 2003, 46, 87-96.] Sequential treatment of aryl aldehyde derivatives of formula **IX** with hydroxylamine hydrochloride, then n-chlorosuccinimide provided intermediates of formula **X**. Aldehyde derivatives of formula **IX** can be obtained commercially, can be prepared by methods known in the literature, or can be readily prepared by one skilled in the art. Intermediates of formula **XI** were prepared by treatment of chlorooximes of formula **XII** with (*E*)-ethyl 3-(pyrrolidin-1-yl)but-2-enoate to afford isoxazoles of formula **XI.** Hydrolysis of the methyl ester of isoxazoles of formula **XI** afforded compounds of formula **VIa**.

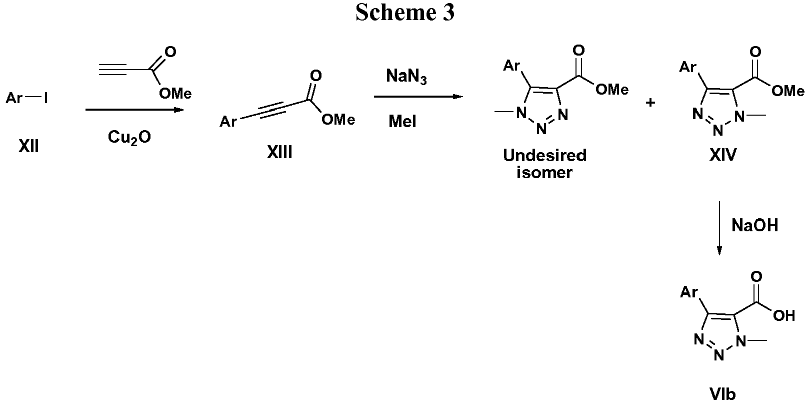

Compounds of formula **VIb** were prepared as outlined in Scheme 3. Aryl iodides of formula **XII** were coupled with methyl propiolate in the presence of copper (I) oxide [See: Liliebris, C.; Larsen, S.D; Ogg, D.; Palazuk, B.J; and Pleasdale, J.E. J.Med.Chem., 2002, 45, 1785.] to provide intermediates of formula **XIII**. Aryl iodide derivatives of formula **XII** can be obtained commercially, can be prepared by methods known in the literature, or can be readily prepared by one skilled in the art. Intermediates of formula **XIII** were treated with sodium azide and methyl iodide to afford triazoles of formula **XIV** after chromatographic separation from an undesired regioisomer. Treatment of triazoles of formula **XIV** with sodium hydroxide provided compounds of formula **VIb**.

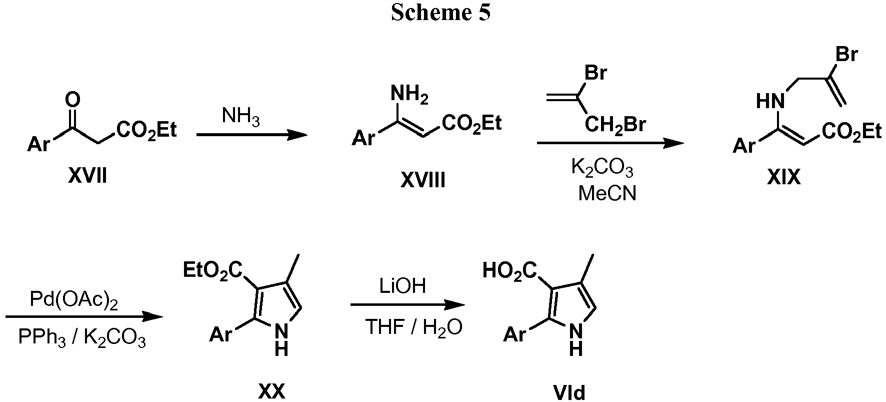

Compounds of formula **VId** were prepared as outlined in Scheme 5 and as described in the literature. [See: Grigg, R.; Savic, V. Chem. Commun. 2000, (10), 873-874.] Beta-ketoesters of formula **XVII** were treated with ammonia to afford enamines of formula **XVIII**. Beta-ketoesters of formula **XVII** can be obtained commercially, can be prepared by methods known in the literature, or can be readily prepared by one skilled in the art. Treatment of enamines of formula **XVIII** with 2,3-dibromoprop-1-ene provided intermediates of formula **XIX**, which upon treatment with palladium(II) acetate afforded pyrroles of formula **XX**. Treatment of pyrroles of formula **XX** with lithium hydroxide provided compounds of formula **VId**.

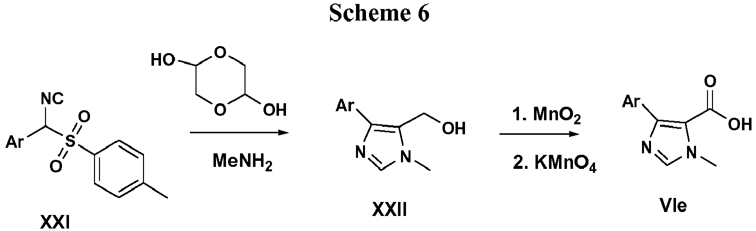

Compounds of formula **VIe** were prepared as outlined in Scheme 6 and as described in the literature. [See: Luke, R.W.A.; Jones, C.D.; McCoull, W; Hayter, B.R. WO Patent 2004013141, 2004**.**] Isocyanates of formula **XXI** were treated with 1,4-dioxane-2,5-diol and methylamine to afford imidazoles of formula **XXII**. Isocyanates of formula **XXI** can be obtained commercially, can be prepared by methods known in the literature, or can be readily prepared by one skilled in the art. Sequential treatment of imidazoles of formula **XXII** with manganese (IV) oxide and potassium permanganate afforded compounds of formula **VIe**.

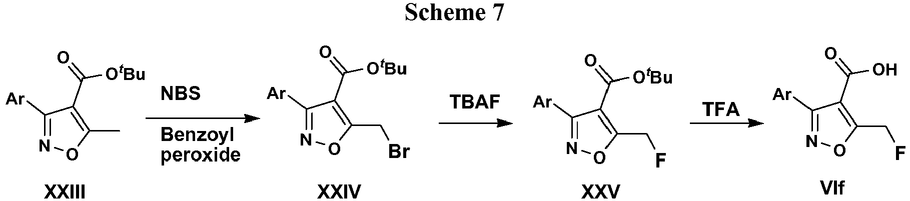

Compounds of formula **VIf** were prepared as outlined in Scheme 7. Isoxazoles of formula **XXIII** were brominated under free radical conditions to provide intermediates of formula **XXIV**. Isoxazoles of formula **XXIV** can be obtained commercially, can be prepared by methods known in the literature, can be prepared by analogy to **Scheme 2**, or can be readily prepared by one skilled in the art. Intermediates of formula **XXIV** were treated with tetrabutylammonium fluoride to afford intermediates of formula **XXV**. [See: Sun, H.; DiMagno, S. G., J.Am. Chem. Soc. 2005, 127, 2050-2051.] Treatment of intermediates of formula **XXV** with trifluoroacetic acid provided compounds of formula **VIf**.

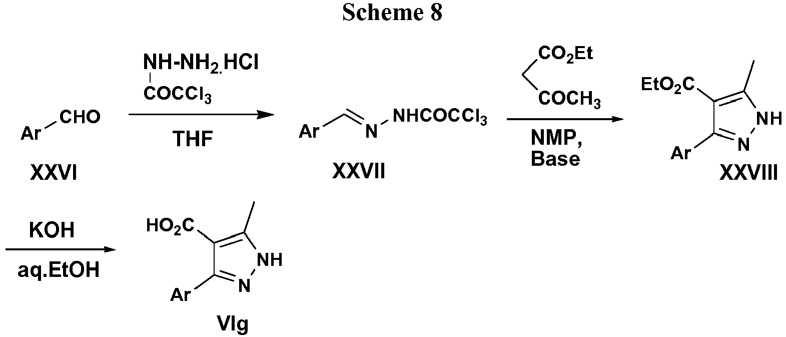

Compounds of formula **VIg** were prepared as outlined in Scheme 8 and as described in the literature. [See: El Kaim,L.; Lacroix, S. Synlett, 2000, 3, 353-354.] Aldehydes of formula **XXVI** were condensed with trichloroacetylhydrazide to afford hydrazones of formula **XXVII**. Aldehydes of formula **XXVI** can be obtained commercially, can be prepared by methods known in the literature, or can be readily prepared by one skilled in the art. Treatment of hydrazones of formula **XXVII** with ethyl acetoacetate under basic conditions afforded pyrazoles of formula **XXVIII**, which were hydrolyzed in a subsequent step to afford compounds of formula **VIg**.

### EXAMPLES

The compounds herein described and set forth and their preparation can be understood further by the following working examples. These examples are meant to be illustrative of the present invention.

Chemical abbreviations used in the Examples are defined as follows:
"Ac" for acetate,
"CD₃OD" for deuteromethanol,
"CDCl₃" for deuterochloroform,
"DCM" for dichloromethane
"DMF" for N,N-dimethylformamide,
"DMAP" for 4-dimethylaminopyridine,
"DMSO" for dimethylsulfoxide,
"EDC" or "EDCI" for 1-(3-dimethylaminopropyl)-3-ethylcarbodiimide hydrochloride,
"Et" for ethyl,
"EtOAC" for ethyl acetate,
"HOAc" for acetic acid,
"NBS" for N-bromosuccinimide,
"NCS" for N-chlorosuccinimide,
"TFA" for trifluoroacetic acid, and
"THF" for tetrahydrofuran.

Abbreviations used in the Examples are defined as follows: "°C" for degrees Celsius, "MS" for mass spectrometry, "ESI" for electrospray ionization mass spectroscopy, "HR" for high resolution, "LC-MS" for liquid chromatography mass spectrometry, "eq" for equivalent or equivalents, "g" for gram or grams, "h" for hour or hours, "mg" for milligram or milligrams, "mL" for milliliter or milliliters, "mmol" for millimolar, "M" for molar, "min" for minute or minutes, "rt" for room temperature, "NMR" for nuclear magnetic resonance spectroscopy, "tlc" for thin layer chromatography, "atm" for atmosphere, and "α", "β", "R", "S", "E", and "Z" are stereochemical designations familiar to one skilled in the art.

"HPLC" is an abbreviation used herein for high pressure liquid chromatography. Reverse-phase HPLC can be carried out using a Vydac C-18 column with gradient elution from 10% to 100 % buffer B in buffer A (buffer A: water containing 0.1% trifluoroacetic acid, buffer B: 10% water, 90% acetonitrile containing 0.1% trifluoroacetic acid). If necessary, organic layers can be dried over sodium sulfate unless otherwise indicated. However, unless otherwise indicated, the following conditions are generally applicable. "LC-MS" refers to high pressure liquid chromatography carried out according to the definition for HPLC with a mass spectrometry detector.

| **Method** | **Start %B** | **Final %B** | **Gradient Time (min)** | **Flow rate (ml/min)** | **λ** | **Column** | **Solvent A** | **Solvent B** |
|---|---|---|---|---|---|---|---|---|
| A | 0 | 100 | 3 | 4 | 220 | Xterra 4.6 X 30mm S5 | 10% MeOH-90% H2O-0.1% TFA | 90% MeOH-10% H2O-0.1% TFA |
| C | 0 | 100 | 3 | 4 | 220 | SunFire C18 5u 4.6 X50mm | 90% Water/ 10% ACN/ 0.1% TFA | 10% Water/ 90% ACN/ 0.1% TFA |
| F | 0 | 100 | 3 | 4 | 220 | Luna 4.6 X 30 mm S10 | 5% ACN - 95% H2O -10mM NH4Ac | 95% ACN-5% H2O-10mM NH4Ac |
| G | 0 | 100 | 4 | 0.8 | 220 | Phenomenex-Luna, 2.0X50mm,3u | 90% H2O-10%ACN-0.1%TFA | 10% H2O-90%ACN -0.1%TFA |
| I | 0 | 100 | 3 | 4 | 220 | Luna 3.0 X 50 mm S10 | 5% ACN - 95% H2O -10mM NH4Ac | 95% ACN-5% H2O-10mM NH4Ac |
| L | 0 | 100 | 2 | 4 | 254 | Phenomenex-Luna 4.6 X 50 mm S10 | 10% MeOH-90% H2O-0.1% TFA | 90% MeOH-10% H2O-0.1% TFA |
| M | 0 | 100 | 2 | 4 | 254 | Phenomenex-Luna 4.6 X 50 mm S10 | 90% Water/ 10% ACN/ 0.1% TFA | 10% Water/ 90% ACN/ 0.1% TFA |
| O | 0 | 95 | 15 | 1.2 | 220 | Ascentis C-18, 4.6 X150 mm | 5% ACN - 95% H2O -10mM NH4Ac | 95% ACN-5% H2O-10mM NH4Ac |
| Q | 0 | 100 | 10 | 2 | 220 | Waters Xbridge C-18 4.6X50 mm | 5% ACN - 95% H2O -10mM NH4Ac | 5% ACN-95% H2O-10mM NH4Ac |
| R | 20 | 95 | 7 | 3 | 220 | Ascentis C-18, 4.6 X50 mm | 5% ACN - 95% H2O -10mM NH4Ac | 95% ACN-5% H2O-10mM NH4Ac |
| U | 30 | 95 | 15 | 20 | 220 | Waters Xbridge C-18 150X19 mm, 5u | H2O -10mM NH4Ac | ACN - 10mM NH4Ac |

Preparatory HPLC: When described as performed under "standard conditions", samples (approx. 20 mg) were dissolved in methanol (10 mg/mL) and purified on a 25mm X 50 mm Vydac C18 column with a 5 minute gradient elution from 10% to 100 % buffer B in buffer A (buffer A: water containing 0.1% trifluoroacetic acid, buffer B: 10% water, 90% acetonitrile containing 0.1% trifluoroacetic acid) at 10 mL/minute.

Melting points were determined on a Mel-Temp II apparatus and are uncorrected. IR spectra were obtained on a single-beam Nicolet Nexus FT-IR spectrometer using 16 accumulations at a resolution of 4.00 cm-1 on samples prepared in a pressed disc of KBr or as a film on KBr plates. Proton NMR spectra (300 MHz, referenced to tetramethylsilane) were obtained on a Varian INOUA 300, Bruker Avance 300, Avance 400, or Avance 500 spectrometer. Data were referred to the lock solvent. Electrospray Ionization (ESI) experiments were performed on a Micromass II Platform single-quadrupole mass spectrometer, or on a Finnigan SSQ7000 mass spectrometer.

### SYNTHESIS OF INTERMEDIATES

### Acid-A. 3-(2-methoxyphenyl)-5-methylisoxazole-4-carboxylic acid

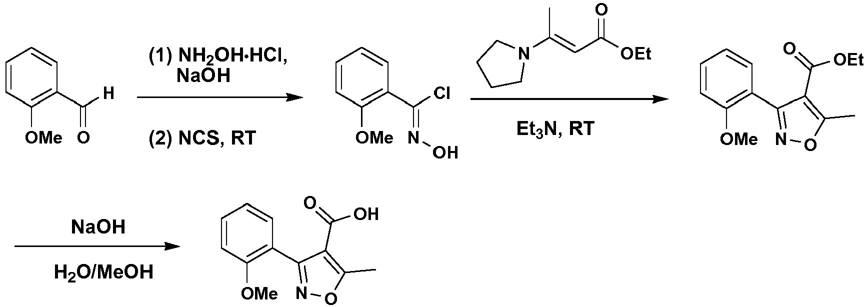

**Step A1**. Ethyl 3-(2-methoxyphenyl)-5-methylisoxazole-4-carboxylate was synthesized from 2-methoxybenzaldehyde as described in [Zamponi, G. W.; Stotz, S. C.; Staples, R. J.; Andro, T. M.; Nelson, J. K.; Hulubei, V.; Blumenfeld, A.; Natale, N. R. J. Med. Chem. 2003, 46, 87-96.]

**Step A2**. The reaction mixture of ethyl 3-(2-methoxyphenyl)-5-methylisoxazole-4-carboxylate (12.85 g, 49.2 mmol) and sodium hydroxide (9.84 g, 246 mmol) in MeOH (100 mL) and Water (10 mL) in a 500-mL round bottom flask was stirred at 65 °C for 20 hours. The MeOH was removed in vacuo, then the concentrated reaction mixture was transferred to a 500-mL separatory funnel with 150 mL of water and 100 mL of ether. The organic layer was discarded. The aqueous layer was acidified by adding concentrated HCl (26 mL). The product precipitated and was separated by filtration and dried under high vacuum to give 10.63 g (93%, theoretical yield 11.47 g). ¹H NMR (400 MHz, CD₃OD) δ 2.69 (s, 3H, C*H*₃), 3.77 (s, 3H, OC*H*₃), 7.00-7.07 (m, 2H, aryl), 7.32-7.34 (m, ¹H, aryl), 7.43-7.48 (m, ¹H, aryl).

The acids below were synthesized by analogy to Acid-A, substituting the appropriate aldehyde for 2-methoxybenzaldehyde.

### Acid-N: 5-methyl-3-(quinolin-8-yl)isoxazole-4-carboxylic acid

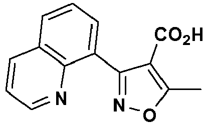

¹H-NMR (DMSO, 400 MHz): δ 8.85 (1H, s), 8.17 (1H, dd, J1 = 7.5 Hz, J2 = 1.5 Hz), 8.14 (1H, dd, J1 = 7.5 Hz, J2 = 1.5 Hz), 7.82 (1H, m),7.69 (1H, s), 7.58 (1H, m), 2.73 (3H, s).

### Acid-S: 5-methyl-3-(3-methylthiophen-2-yl)isoxazole-4-carboxylic acid

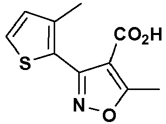

¹H-NMR (DMSO, 400 MHz): δ 7.49-7.74 (dd, 1H), 6.96-7.24(dd, 1H), 2.7 (s, 3H), 2.13 (s,3H). m/e 224(M+1)⁺.

### Acid-X: 5-methyl-3-(naphthalen-1-yl)isoxazole-4-carboxylic acid

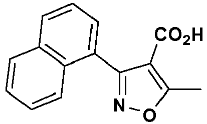

¹H-NMR (DMSO, 400 MHz): δ 7.83-8.22 (m, 3H), 7.4-7.7 (m, 4H), 3.5 (2H, bs), 2.74 (3H, s). m/e 253.97 (M+1)⁺.

### Acid-Z: 3-(isoquinolin-5-yl)-5-methylisoxazole-4-carboxylic acid

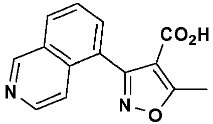

¹H-NMR (DMSO, 400 MHz): δ 9.14-9.49 (1H, m), 8.37-8.47 (1H, m), 8.24-8.37(1H, m), 7.76-7.98 (2H, m),7.52-7.72 (1H, m), 2.82 (3H, s). m/e 254.80 (M+1)⁺.

### Acid-AH: 3-(2-chloropyridin-3-yl)-5-methylisoxazole-4-carboxylic acid

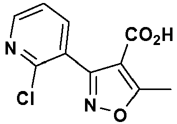

¹H-NMR (CD₃OD, 500 MHz): δ 8.52-8.44 (1H, m), 7.92-7.83 (1H, m), 7.53-7.44 (1H, m), 2.76 (3H, s).

### Acid-AI: 5-methyl-3-(quinoxalin-5-yl)isoxazole-4-carboxylic acid

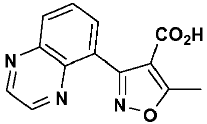

¹H-NMR (DMSO, 500 MHz): δ 12.62 (1H, s), 9.01 (1H, d, *J* = 1.8Hz), 8.91 (1H, d, *J* = 1.5 Hz), 8.28-8.21 (1H, m), 7.99-7.92 (2H, m), 2.74 (3H, s).

### Acid-AJ: 3-(2-methoxynaphthalen-1-yl)-5-methylisoxazole-4-carboxylic acid

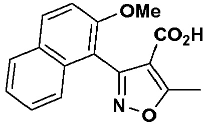

¹H-NMR (DMSO, 400 MHz): δ 12.60 (1H, s), 8.09 (1H, d, *J* = 9.0 Hz), 7.93 (1H, d, *J* = 7.8 Hz), 7.55 (1H, d, *J* = 9.0 Hz), 7.46-7.33 (3H, m), 3.84 (3H, s), 2.77 (3H, s).

### Acid-AK: 5-(fluoromethyl)-3-(2-methoxyphenyl)isoxazole-4-carboxylic acid

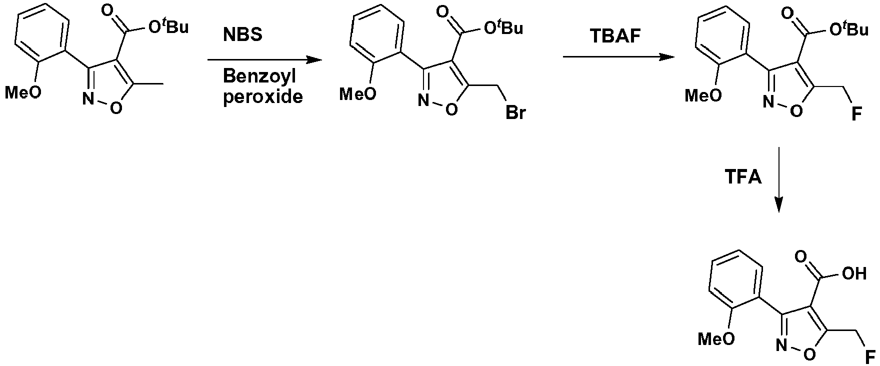

**Step AK1.** A solution of *tert*-butyl 3-(2-methoxyphenyl)-5-methylisoxazole-4-carboxylate (0.795 g, 2.75 mmol), NBS (0.978 g, 5.50 mmol) and benzoyl peroxide (0.033 g, 0.137 mmol) in CCl₄ (10 mL) was stirred at 90 °C for 16 hours. The reaction mixture was cooled to RT and the solid was filtered off. Solvent was removed in *vacuo*. The product was purified by flash chromatography (DCM, R*f* 0.56) to give 0.44 g (44% yield). ¹H-NMR (CDCl₃, 400 MHz): δ 7.49-7.37 (2H, m), 7.04 (1H, t, *J* = 7.5 Hz), 6.96 (1H, d, *J* = 8.3 Hz), 4.79 (2H, s), 3.79 (3H, s), 1.38 (9H, s).

**Step AK2.** *tert*-butyl 5-(fluoromethyl)-3-(2-methoxyphenyl)isoxazole-4-carboxylate was prepared from *tert*-Butyl 5-(bromomethyl)-3-(2-methoxyphenyl)isoxazole-4-carboxylate by the method described in Sun, H.; DiMagno, S. G., J.Am. Chem. Soc. 2005, 127, 2050-2051. ¹H-NMR (CDCl₃, 400 MHz): δ 7.49-7.37 (2H, m), 7.08-7.01 (1H, m), 6.97 (1H, d, *J* = 8.3 Hz), 5.72 (2H, d, *J* = 47.2 Hz), 3.79 (3H, s), 1.35 (9H, s).

**Step AK3.** Treating *tert*-butyl 5-(fluoromethyl)-3-(2-methoxyphenyl)isoxazole-4-carboxylate with TFA/DCM (1:1) at room temperature for one hour followed by evaporation of DCM/TFA in *vacuo* provided the title compound. ¹H-NMR (CD₃OD, 400 MHz): δ 7.53-7.44 (1H, m), 7.42-7.35 (1H, m), 7.13-6.98 (2H, m), 5.74 (2H, d, *J* = 47.2 Hz), 3.78 (3H, s).

### Acid-AO: 2-(2-Chlorophenyl)-4-methyl-1H-pyrrole-3-carboxylic acid

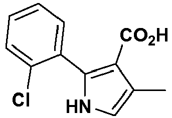

The title compound was prepared according to the literature procedure: Grigg, R.; Savic, V. Chem. Commun. 2000, (10), 873-874. ¹H-NMR (CDCl₃, 400 MHz): δ 7.52-7.63(1H, m), 7.41-7.48 (2H, m), 7.28-7.32 (1H,m), 6.8-6.85 (1H, m), 2.17 (3H, s).

### Acid-AT: 5-(2-methoxyphenyl)-3-methyl-1H-pyrazole-4-carboxylic acid

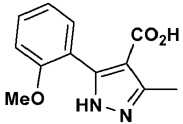

The title compound was prepared according to the literature procedure: El Kaim,L.; Lacroix, S. Synlett, 2000, 3, 353-354. ¹H-NMR (CDCl₃, 400 MHz): δ 7.28-7.40 (1H, m), 7.17-7.25 (1H,m), 6.90-7.08 (2H, m), 2.38 (3H, s). m/e 233.23 (M+1)⁺.

### Acid-AU: 4-(2-methoxyphenyl)-1-methyl-1H-1,2,3-triazole-5-carboxylic acid

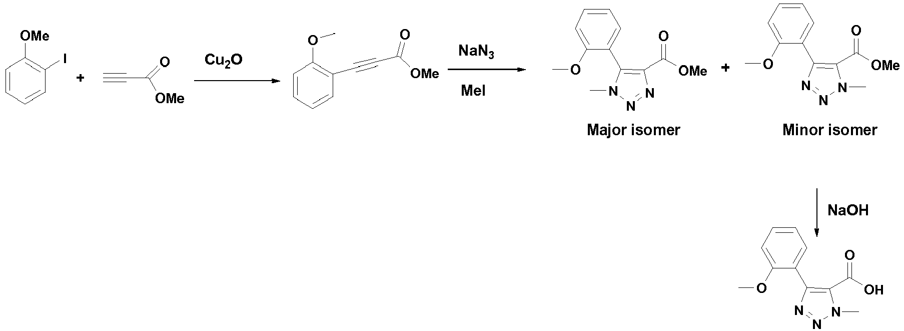

**Step AU1.** Reference: Liliebris, C.; Larsen, S.D; Ogg, D.; Palazuk, B.J; and Pleasdale, J.E. J.Med.Chem., 2002, 45, 1785.

To a suspension of methyl propiolate (**2**, 1.314 ml, 15.41 mmol) and copper(I) oxide (1.086 g, 7.59 mmol) in DMF (20 ml) was added 1-iodo-2-methoxybenzene (**1**, 1.262 ml, 9.48 mmol). The resulting mixture was heated in a Microwave reactor to 110 °C for 2hrs. The reaction mixture was filtered through a short pad of silica gel and washed w/ EtOAc. The organic layer was washed w/1M HCl, brine and sat'd NaHCO₃, dried (Na₂SO₄) and concentrated to leave an oil as crude product, which was purified by flash chromatography (SiO₂, EtOAc/Hexane, 1:2) to afford methyl 3-(2-methoxyphenyl)propiolate **3** (932mg, 4.66 mmol, 49.1 % yield). ¹H-NMR(500MHz, CDCl3), δ : 7.54(1H, dd), 7.44(1H, td), 6.98-6.92(m,2H), 3.92(s,3H), 3.86(s,3H).

**Step AU2.** A mixture of methyl 3-(2-methoxyphenyl)propiolate (**3**, 932 mg, 4.90 mmol), sodium azide (478 mg, 7.35 mmol), and iodomethane (0.458 ml, 7.35 mmol) in Water (7 ml) and DMF(3 ml) was heated to 100 °C in a Microwave reactor for 6hrs. Purified by Prep-HPLC (Varian, 15-90B in min., B=90% MeOH/10% H2O) to afford methyl 5-(2-methoxyphenyl)-1-methyl-1*H*-1,2,3-triazole-4-carboxylate (701 mg, 2.69 mmol, 55.0 % yield), ¹H-NMR(500MHz, CD3OD), δ : 7.52-7.56(m, 1H), 7.30 (dd, 1H), 7.12, (td, 1H), 7.06, (d,1H), 3.90 (s, 3H), 3.86(s,3H), 3.82(s, 3H). and methyl 4-(2-methoxyphenyl)-1-methyl-1H-1,2,3-triazole-5-carboxylate (185 mg, 0.711 mmol, 14.51 % yield), ¹H-NMR( 500MHz, CD3OD), δ : 7.49-7.46(m,2H), 7.10-7.07(m,2H), 4.32(s,3H), 3.79(s, 3H), 3.78(s,3H).

**Step AU3.** To a soln. of methyl 4-(2-methoxyphenyl)-1-methyl-1H-1,2,3-triazole-5-carboxylate (183 mg, 0.74 mmol) in MeOH (5ml) was added sodium hydroxide (1.3 ml, 3M aq. solution). The mixture was stirred at r.t for 2hrs, then concentrated in vacuo. The residue was taken up in water, washed w/ether (3x) to remove the possible impurity. The aq. phase was acidified w/6M HCl to pH 3, extracted w/EtOAc (4x). The combined organic layer was dried and evaporated to afford the title compound (164mg, 0.703 mmol, 95 % yield). ¹H-NMR(500MHz, CD3OD), δ : 7.46-7.42(m,2H), 7.09-7.05(m,2H), 4.32(s,3H), 3.73(s,3H).

### Acid-AV: 4-(2-methoxyphenyl)-1-methyl-1H-imidazole-5-carboxylic acid

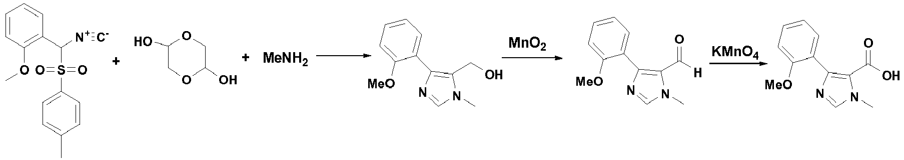

Reference: Luke, R.W.A.; Jones, C.D.; McCoull, W; Hayter, B.R. WO Patent 2004013141, 2004**.**

**Step AV1.** To a soln. of 1,4-dioxane-2,5-diol (120 mg, 0.995 mmol) in THF (8ml) was added methylamine (2.8 ml, 0.664 mmol) at r.t. The resulting mixture was stirred at r.t for 75min.

Then 1-(isocyano(tosyl)methyl)-2-methoxybenzene (200 mg, 0.664 mmol) was added while keeping reaction mixture at <30 °C by a water bath. The reaction mixture was stirred at r.t overnight. Evaporated to leave white solid, dissolved in DMF, and purified by Pre-HPLC to afford (4-(2-methoxyphenyl)-1-methyl-1H-imidazol-5-yl)methanol (84mg, 0.377 mmol, 38.6 % yield) as a colorless oil. ¹H-NMR(MeOD), δ: 8.97(1H,s), 7.55(1H,t, J=7.5Hz), 7.47(1H, d, J=8.0Hz), 7.22(1H, d, 8.0Hz), 7.15(1H, t, J=7.5Hz), 4.67(2H,s), 4.05(3H,s), 3.89(3H,s).

**Step AV2.** To a soln. of (4-(2-methoxyphenyl)-1-methyl-1H-imidazol-5-yl)methanol (84mg, 0.385 mmol) in 1,4-Dioxane (5ml) was added MnO2 (147 mg, 1.693 mmol). The mixture was heated to 90 °C for 4hrs or until LC/MS showed the completion of the reaction. The reaction mixture was Filtered through celite, and evaporated to afford 4-(2-methoxyphenyl)-1-methyl-¹H-imidazole-5-carbaldehyde (78mg, 0.325 mmol, 84 % yield), which was used for the next reaction w/o purification.

**Step AV3.** To a soln. of 4-(2-methoxyphenyl)-1-methyl-1H-imidazole-5-carbaldehyde (78 mg, 0.361 mmol) in acetone (5ml) and water (1 ml) was added potassium carbonate (100 mg, 0.721 mmol). After potassium was dissolved, KMnO4 (123 mg, 0.776 mmol) was added at r.t. The mixture was stirred for 24hrs. LC/MS showed the completion. The mixture was filtered through celite, washed w/water. The actone was evaporated from the filtrate which was extracted with EtOAc (2x). The aq. layer was acidified w/ HOAc to PH=5, reduced the volume to half volume, freezed and lyphilized to leave solid, which was purified by Pre-HPLC to afford the title compound (41 mg, 0.173 mmol, 48 % yield). ¹H-NMR(MeOD), δ: 8.06(s,1H), 7.41-7.36(m, 2H), 7.05-6.99(m, 2H), 3.98(s,3H), 3.80(s,3H).

Methyl 1-(2-chlorophenyl)-4-methyl-¹H-1,2,3-triazole-5-carboxylate (methyl ester of **Acid-AX**) was prepared as described in [Bell, M. G. et al. PCT Int. Appl. 2007, WO 2007140174.] The methyl ester was hydrolyzed (NaOH/ H2O/ MeOH, RT) to afford the title compound. NMR for methyl ester of **Acid-AX:** ¹H-NMR (CD₃OD, 400 MHz): δ 7.67-7.58 (2H, m), 7.55-7.51 (2H, m), 3.77 (3H, s), 2.61 (3H, s).
NMR for **Acid-AX:** ¹H-NMR (CD₃OD, 400 MHz): δ 7.65-7.50 (4H, m), 2.61 (3H, s).

### Amine-B: 1-(3-bromo-5-nitropyridin-2-yl)piperazine

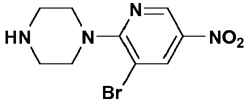

**Step B1**. A solution of 3-bromo-2-chloro-5-nitropyridine (0.475 g, 2.000 mmol), tert-butyl piperazine-1-carboxylate (0.373 g, 2 mmol), and triethylamine (0.405 g, 4.00 mmol) in DCM (10 mL) was stirred at RT overnight. Purified by silicon gel column with 5% EtOAc in DCM gave tert-butyl 4-(3-bromo-5-nitropyridin-2-yl)piperazine-1-carboxylate (0.68 g, 1.756 mmol, 88 % yield). yellow solid. ¹H-NMR(CDCl₃, 500MHz) δ9.02 (1H, s), 8.55 (1H, s), 3.59-3.65 (8H,m), 1.50(9H,s).

**Step B2.** tert-butyl 4-(3-bromo-5-nitropyridin-2-yl)piperazine-1-carboxylate was treated with 30% TFA in DCM (5ml) for 2 hours. Drained and dried to afford the title compound (0.71 g, 1.770 mmol, 88 % yield) as a yellow solid. ¹H-NMR(CD₃OD, 500MHz) δ 9.08 (1H, s), 8.72 (1H, s), 3.85 (4H,m), 3.41 (4H,m).

### Amine E: (3-(2-chlorophenyl)-5-methylisoxazol-4-yl)(piperazin-1-yl)methanone

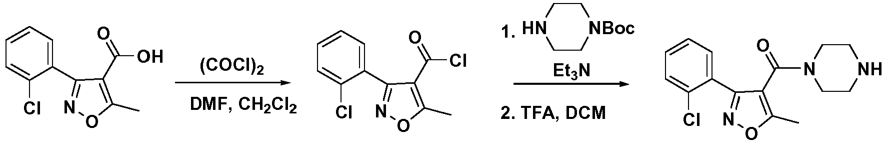

**Steps E1**-**E2**. To a solution of 3-(2-chlorophenyl)-5-methylisoxazole-4-carboxylic acid (2.376 g, 10.00 mmol) in DCM (70 mL) was added 2 drops of DMF, then oxalyl dichloride (1.523 g, 12.00 mmol) in portions. The mixture was stirred for 2 hours and the solution turn clear. The solvent was removed by rotovap and the residue was dried in vacuum for 10 min. The residue was dissolved in DCM (70 mL), then triethylamine (3.04 g, 30.0 mmol) and tert-butyl piperazine-1-carboxylate (1.862 g, 10.00 mmol) was added, the resulting mixture was stirred overnight. After the solvent was removed by rotovap, the residue was purified by silicon gel column with DCM, then 10% EtOAc in DCM to give tert-butyl 4-(3-(2-chlorophenyl)-5-methylisoxazole-4-carbonyl)piperazine-1-carboxylate (3.6 g, 8.87 mmol, 89 % yield).

**Step E3.** tert-butyl 4-(3-(2-chlorophenyl)-5-methylisoxazole-4-carbonyl)piperazine-1-carboxylate was treated with 50% TFA in DCM (20ml) for 2 hours. After the solvent was removed, the residue was purified by silicon gel column with 10% MeOH in DCM to give (3-(2-chlorophenyl)-5-methylisoxazol-4-yl)(piperazin-1-yl)methanone (2.4g, 7.85 mmol, 79 % yield). ¹H-NMR(CD₃OD, 500MHz) δ 7.54-7.61(3H, m.), 7.50(1H, t, J =7.3Hz), 3.63 (4H,s), 2.94 (4H,s), 2.58 (3H,s).

### Example 1

### (3-(2-chlorophenyl)-5-methylisoxazol-4-yl)(4-(2-methyl-4-nitrophenyl)piperazin-1-yl)methanone

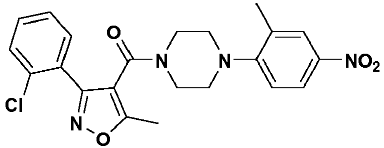

A mixture of 3-(2-chlorophenyl)-5-methylisoxazole-4-carboxylic acid (0.050 g, 0.210 mmol), 1-(2-methyl-4-nitrophenyl)piperazine (0.071 g, 0.210 mmol) (TFA salt), EDC (0.048 g, 0.252 mmol) and DMAP (0.051 g, 0.421 mmol) in dichloromethane (3 mL) was stirred at room temperature overnight. The solvent was evaporated *in vacuo*. The crude product was purified by preparative HPLC (methanol/ water with 0.1% TFA) to give 41 mg (44% yield) of the title compound. ¹H-NMR (300 MHz, CDCl₃) δ 8.08-7.99 (2 H, m), 7.60-7.36 (4 H, m), 6.81 (1 H, d, *J*=8.4 Hz), 3.79 (2 H, br. s.), 3.39 (2 H, br. s.), 2.89 (2 H, br. s.), 2.61 (3 H, s), 2.40 (2 H, br. s.), 2.33 (3 H, s). HPLC/MS (Method U): (ES+) m/z (M+H)⁺ = 441; Rₜ = 5.67 min.

### Example 5

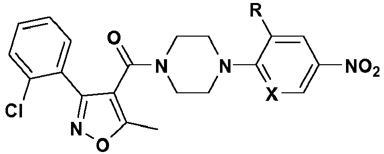

### Example 5 was synthesized by analogy to Example 1, substituting the appropriate RHS Preparation for 1-(2-methyl-4-nitrophenyl)piperazine.

| **Example** | **R** | **X** | **MH+** | **RT** | **LC/MS Method** | **RHS Preparation** |
|---|---|---|---|---|---|---|
| **5** | Br | N | 508 | 1.60 | M | Amine-B |

### Examples 10 and 18

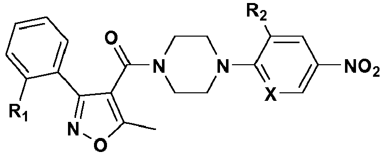

### Examples 10 and 18 were synthesized by analogy to Example 1, substituting the appropriate RHS Preparation for 1-(2-methyl-4-nitrophenyl)piperazine and the appropriate LHS Preparation for 3-(2-chlorophenyl)-5-methylisoxazole-4-carboxylic acid.

| **Example** | **R₁** | **R₂** | **X** | **MH+** | **RT** | **LC/MS Method** | **LHS Prep.** | **RHS Prep.** |
|---|---|---|---|---|---|---|---|---|
| **10** | OCH₃ | Br | N | 504 | 2.28 | L | Acid-A | Amine-B |
| **18** | OCH₃ | Cl | CH | 457 | 2.92 | A | Acid-A | Commercial |

### Examples 39, 44, 49, 51, 61, 62, 63

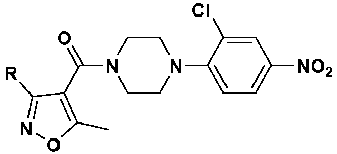

### These Examples were synthesized by analogy to Example 1, substituting 1-(2-chloro-4-nitrophenyl)piperazine for 1-(2-methyl-4-nitrophenyl)piperazine and the appropriate LHS Preparation for 3-(2-chlorophenyl)-5-methylisoxazole-4-carboxylic acid.

| **Example** | **R** | **MH+** | **RT** | **LC/MS Method** | **LHS Prep** |
|---|---|---|---|---|---|
| **39** | quinolin-8-yl | 479 | 8.96 | O | Acid-N |
| **44** | 3-methylthiophen-2-yl | 447 | 5.39 | Q | Acid-S |
| **49** | naphth-1-yl | 477 | 4.88 | Q | Acid-X |
| **51** | isoquinolin-5-yl | 478 | 4.55 | Q | Acid-Z |
| **61** | 2-chloropyrid-3-yl | 462 | 2.74 | A | Acid-AH |
| **62** | quinoxalin-5-yl | 479 | 1.76 | I | Acid-AI |
| **63** | 2-methoxynaphth-1-yl | 507 | 2.48 | C | Acid-AJ |

### Example 67

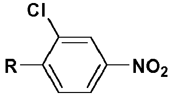

### Example 67 was synthesized by analogy to Example 1, substituting the appropriate RHS Preparation for 1-(2-methyl-4-nitrophenyl)piperazine and the appropriate LHS Preparation for 3-(2-chlorophenyl)-5-methylisoxazole-4-carboxylic acid.

| **Example** | **R** | **MH+** | **RT** | **LC/MS Method** | **LHS Prep.** | **RHS Prep.** |
|---|---|---|---|---|---|---|
| **67** | 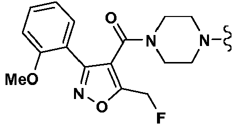 | 475 | 2.30 | C | Acid-AK | Commer cial |

### Examples 75, 80, 81, 82

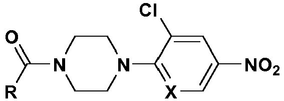

### These Examples were synthesized by analogy to Example 1, substituting the appropriate RHS Preparation for 1-(2-methyl-4-nitrophenyl)piperazine and the appropriate LHS Preparation for 3-(2-chlorophenyl)-5-methylisoxazole-4-carboxylic acid.

| **Example** | **X** | **R** | **MH+** | **RT** | **LC/MS Method** | **LHS Prep.** | **RHS Prep.** |
|---|---|---|---|---|---|---|---|
| **75** | CH | 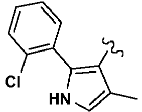 | 459 | 8.09 | O | Acid-AO | Commercial |
| **80** | CH | 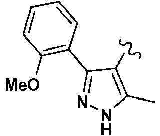 | 456 | 3.59 | R | Acid-AT | Commercial |
| **81** | CH | 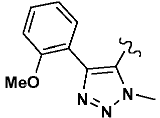 | 457 | 2.97 | G | Acid-AU | Commercial |
| **82** | CH | 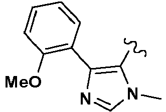 | 456 | 2.24 | G | Acid-AV | Commercial |

### Example 96 (Reference only)

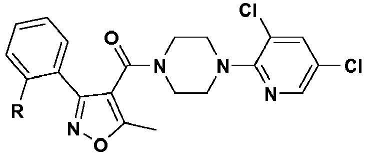

### Example 96 was synthesized by analogy to Example 1, substituting 1-(3,5-dichloro-piperidin-2-yl)piperazine for 1-(2-methyl-4-nitrophenyl)piperazine and the appropriate LHS Preparation for 3-(2-chlorophenyl)-5-methylisoxazole-4-carboxylic acid.

| **Example** | **R** | **MH+** | **RT** | **LC/MS Method** | **LHS Prep.** |
|---|---|---|---|---|---|
| **96** | OCH₃ | 447 | 2.20 | F | Acid-A |

### Example 142 (Reference only)

### (4-(3,5-dichloropyridin-2-yl)piperazin-1-yl)(3-(2-hydroxyphenyl)-5-methylisoxazol-4-yl)methanone

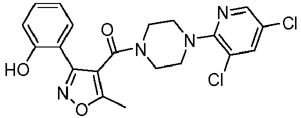

A 1.0 M solution of boron tribromide (5.70 mL, 5.70 mmol) was cooled to -78 °C. A solution of (4-(3,5-dichloropyridin-2-yl)piperazin-1-yl)(3-(2-methoxyphenyl)-5-methylisoxazol-4-yl)methanone (**Example 96**, 0.85 g, 1.900 mmol) in DCM (10 mL) was added dropwise. The reaction mixture was stirred at -78 °C for 3 hr. and at room temperature overnight. Water was added slowly, followed by the addition of saturated NaHCO₃ solution. The product was extracted with DCM (3 X 50 mL). The crude product was purified via column chromatography (20% EtOAc/ DCM R_{f}0.45) to give 0.75 g (92%, theoretical yield 0.823 g) of the title compound. ¹H-NMR (CDCl₃, 300 MHz): δ 9.31 (1H, s), 8.12 (1H, d, *J* = 2.2 Hz), 7.62 (1H, d, *J* = 2.2 Hz), 7.50-7.40 (1H, m), 7.40-7.30 (1H, m), 7.14-7.06 (1H, m), 6.99-6.87 (1H, m), 3.98 (2H, t, *J* = 5.1 Hz), 3.40 (2H, t, *J* = 5.1 Hz), 3.35 (2H, s), 3.05 (2H, s), 2.54 (3H, s). HPLC/MS (Method F): (ES+) m/z (M+H)⁺ = 433; Rₜ = 2.05 min.

### Example 147

### (4-(2-chloro-4-nitrophenyl)piperazin-1-yl)(3-(2-hydroxyphenyl)-5-methylisoxazol-4-yl)methanone

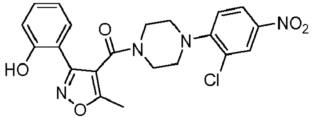

The title compound was prepared by analogy to **Example 142**, substituting **Example 18** for **Example 96**. ¹H-NMR (CDCl₃, 500 MHz): δ 9.20 (1H, s), 8.26 (1H, d, *J* = 2.7 Hz), 8.10 (1H, dd, *J*1 = 9.0 Hz, *J*2 = 2.7 Hz), 7.47-7.42 (1H, m), 7.40-7.34 (1H, m), 7.12 (1H, d, *J* = 8.6 Hz), 6.99-6.90 (2H, m), 4.03 (2H, s), 3.37 (2H, s), 3.23 (2H, s), 2.69 (2H, s), 2.56 (3H, s). HPLC/MS (Method C): (ES+) m/z (M+H)⁺ = 443; Rₜ = 2.16 min.

### Materials and Methods

### Cells and Virus

Madin Darby canine kidney (MDCK) cells and influenza A/WSN/33 were obtained from ATCC. Influenza A/Solomon Islands/3/06 and influenza A/Brisbane/10/2007 were obtained from the CDC.

### Compounds

Test compounds, at 100x the final test concentration, were serially diluted in DMSO in 3-fold steps. One ul of diluted compound was added to each well of a 96-well plate.

### Antivial Assays

For antiviral assays, MDCK cells were re-suspended in assay media (MEM with pen/strep plus 0.125% BA (bovine albumin) and 1 ug/ml TPCK-treated trypsin) at 4.5 x 10⁵ cells per ml. Virus was added for final multiplicity of infection (MOI) of 0.001 plaque forming units per cell and 100 ul was added to each well of a 96-well plate (lul of compound/well). For cytotoxicity assays, only cells were added to the assay plates. 48 hrs post infection, viral replication in the presence of inhibitor was determined by measuring viral neuraminidase (NA) activity via activation of the quenched substrate 2'-(4-Methylunbelliferyl)-α-D-N-acetylneuraminic acid (MUNANA). A 5x substrate solution was added to yield a final concentration of 100 uM MUNANA, 50 mM MES, 2 mM CaCl₂ and 0.25% NP-40. After a 30 minute incubation at 37 °C the plates were read on a fluorescence plate reader set at 360 nm excitation and 460 nm emission. Cytotoxicity was ascertained via crystal violet staining of treated cells. Cells were washed once with PBS, stained for 20 min with 0.5% crystal violet in 20% methanol, washed with water and air dried. 50ul of methanol was added to each well to solubilize the crystal violet and 50 ul PBS was added before the absorbance was read at 540 nM.

### References

Chen J, Deng YM. 2009. Influenza virus antigenic variation, host antibody production and new approach to control epidemic. Virol J. Mar 13;6:30.
Deyde VM, Sheu TG, Trujillo AA, Okomo-Adhiambo M, Garten R, Klimov AI, Gubareva LV. 2010. Detection of molecular markers of drug resistance in 2009 pandemic influenza A (H1N1) viruses by pyrosequencing. Antimicrob Agents Chemother. Mar;54(3):1102-10.
Moscona A. 2009. Global transmission of oseltamivir-resistant influenza. N Engl J Med. Mar 5;360(10):953-6.
Soepandi PZ, Burhan E, Mangunnegoro H, Nawas A, Aditama TY, Partakusuma L, Isbaniah F, Malik S, Benamore R, Baird JK, Taylor WR. 2010. Clinical course of H5N1 avian influenza in patients at the Persahabatan Hospital, Jakarta, Indonesia, 2005-2008. Chest 09-2644.
Zimmer SM, Burke DS. 2009. Historical perspective--Emergence of influenza A (H1N1) viruses. N Engl J Med. Jul 16;361(3):279-85.

**Activity Table 1**

| **Example** | **A/H1N1/WSN** | **A/H1N1/ Solomon Islands** | **A/H3N2 Brisbane** | **A/H5N1/Duck MN** | **A/H5N1/Duck_PA** | **A/H5N1/Gull_PA** |
|---|---|---|---|---|---|---|
| | + | + | - | + | + | ++ |
| **82** | ++ | - | - | + | + | ++ |
| **18** | +++ | ++ | + | ++ | ++ | +++ |
| **81** | +++ | + | - | ++ | ++ | +++ |
| **75** | +++ | + | - | ++ | ++ | +++ |
| **61** | +++ | + | - | ++ | ++ | ++ |
| **63** | +++ | ++ | - | ++ | ++ | +++ |

| | | | | | | |
|---|---|---|---|---|---|---|
| Table Key: "-" = EC₅₀ > 10 uM; "+" = EC₅₀ ≤10 uM; "++" = EC₅₀ < 1 uM; "+++" = EC₅₀ < 0.1 uM | | | | | | |

**Activity Table 2**

| **Example** | **Human Liver Microsomal Stability (%Remaining after 10 minutes)** | **Mouse Liver Microsomal Stability (%Remaining after 10 minutes)** |
|---|---|---|
| 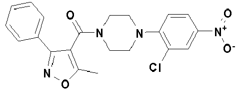 | 40 | 20 |
| (comparative) | | |
| 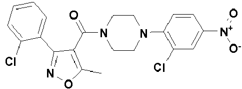 | 6 | 0 |
| (comparative) | | |
| **81** | 73 | 45 |
| **80** | 72 | 65 |
| **61** | 50 | 18 |
| **82** | 45 | 43 |
| **67** | 40 | 29 |

## Claims

1. A compound which is selected from the group consisting of: 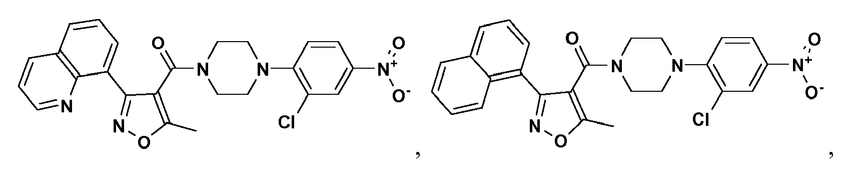 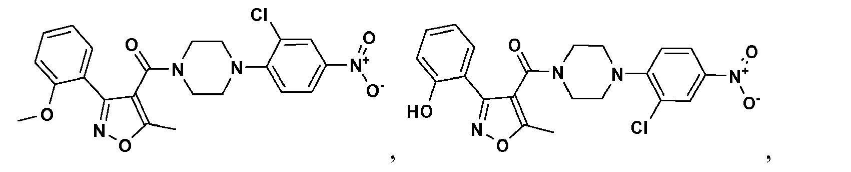 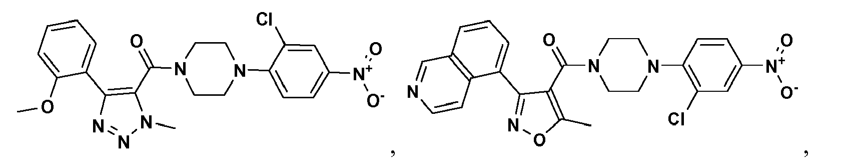 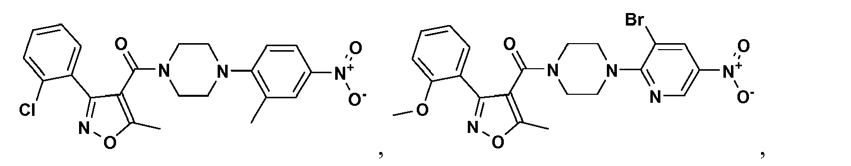 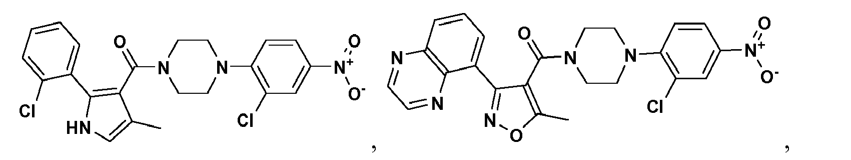 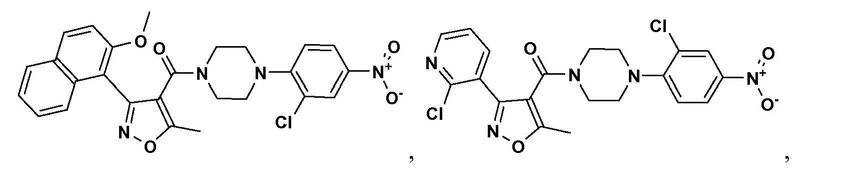 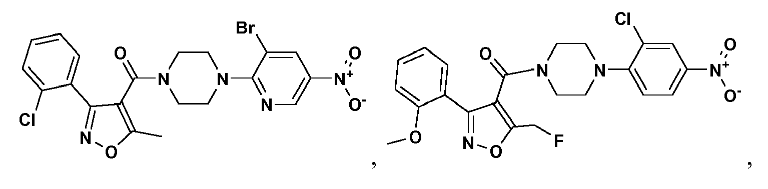 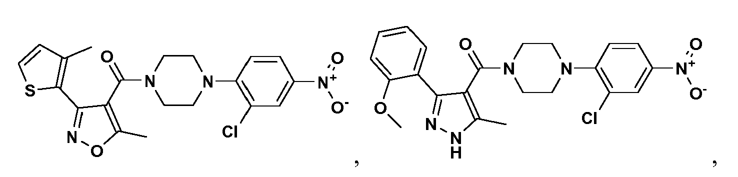 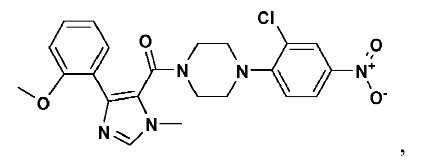 and pharmaceutically acceptable salts thereof.

2. A pharmaceutical composition which comprises an antiviral effective amount of one or more of the compounds of claim 1, together with one or more pharmaceutically acceptable carriers, excipients or diluents.

3. A compound of claim 1, and one or more pharmaceutically acceptable carriers, excipients or diluents for use in treating a mammal infected with influenza virus.

## Patentansprüche

1. Verbindung, die ausgewählt ist aus der Gruppe bestehend aus: 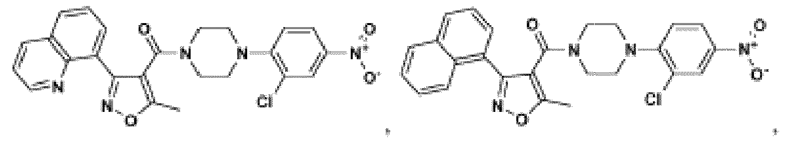 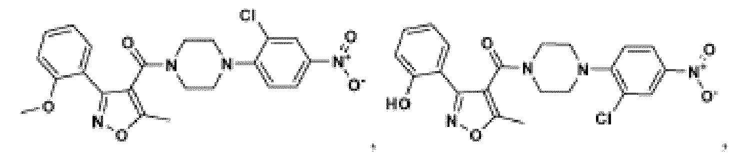 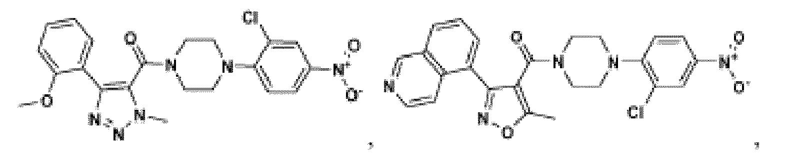 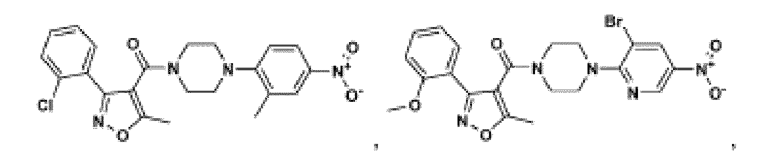 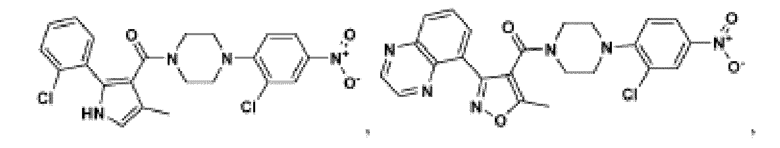 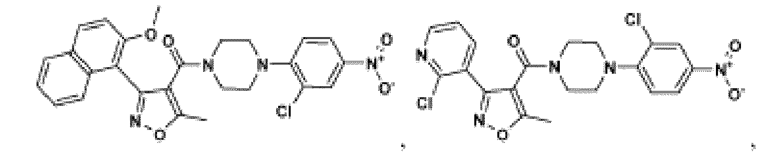 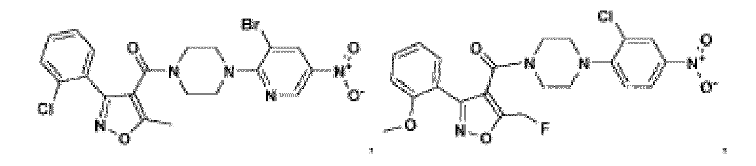 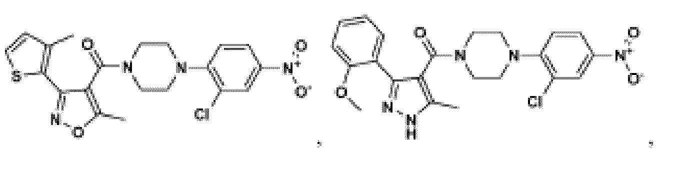 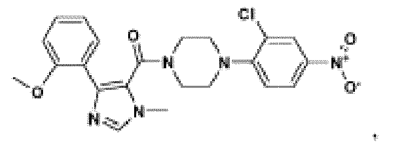 und pharmazeutisch verträgliche Salze davon.

2. Pharmazeutische Zusammensetzung, die eine antivirale wirksame Menge einer oder mehrerer der Verbindungen nach Anspruch 1, zusammen mit einem oder mehreren pharmazeutisch verträglichen Trägern, Hilfsstoffen oder Verdünnungsmitteln, umfasst.

3. Verbindung nach Anspruch 1, und ein oder mehrere pharmazeutisch verträgliche Träger, Hilfsstoffe oder Verdünnungsmittel, zur Anwendung bei der Behandlung eines mit dem Influenzavirus infizierten Säugers.

## Revendications

1. Composé sélectionné à partir du groupe constitué de: 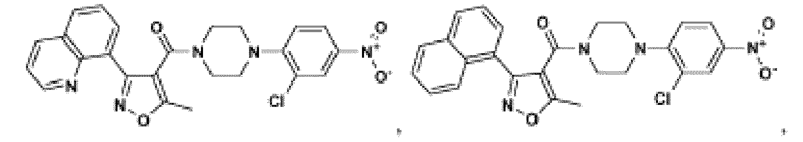 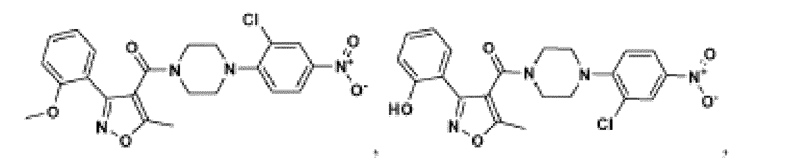 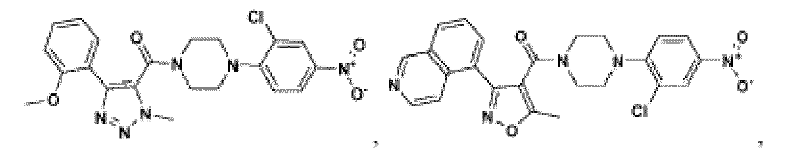 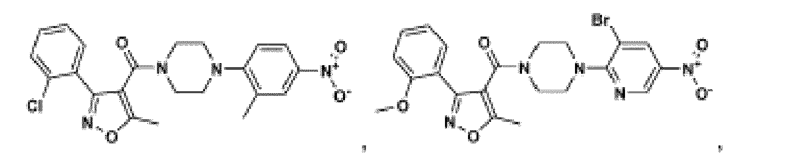 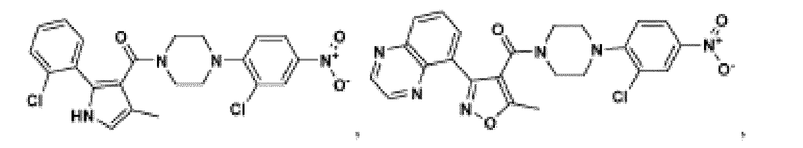 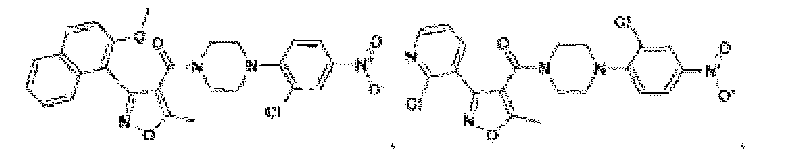 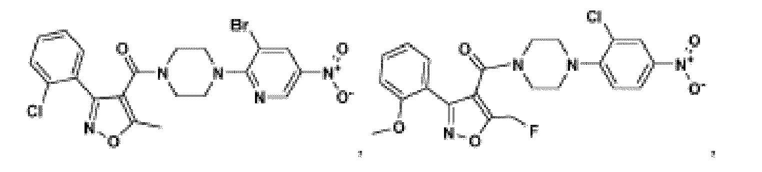 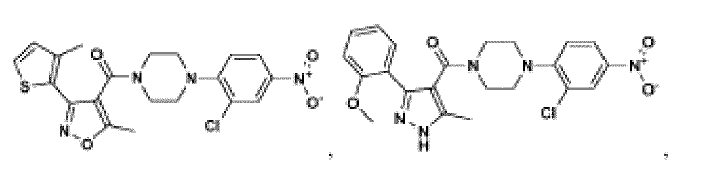 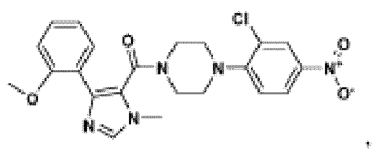 et de sels pharmaceutiquement acceptables de celui-ci.

2. Composition pharmaceutique comprenant une quantité effective antivirale d'un ou plusieurs composés selon la revendication 1, conjointement avec un ou plusieurs supports, excipients ou diluants pharmaceutiquement acceptables.

3. Composé selon la revendication 1 et un ou plusieurs supports, excipients ou diluants pharmaceutiquement acceptables pour une utilisation dans le traitement d'un mammifère infecté par le virus de la grippe.
